# EUROPÄISCHE PATENTSCHRIFT

(11) **EP 1 970 088 B1**
(45) Veröffentlichungstag und Bekanntmachung des Hinweises auf die Patenterteilung: **18.11.2009**
(21) Anmeldenummer: 07005470.5
(22) Anmeldetag: 16.03.2007
(51) Int. Cl.: A61M 25/00

(54) **Katheter mit volumenveränderlichen Abschnitten**
Catheter with sections with changeable volume
Cathéter doté de parties à volume variable

(43) Veröffentlichungstag der Anmeldung: 17.09.2008
(73) Patentinhaber: BrainLAB AG, 85622 Feldkirchen (DE)
(72) Erfinder: Mittermeyer, Stephan, 84036 Landshut (DE); Hartlep, Andreas, Dr., 83629 Weyarn (DE)
(74) Vertreter: Schwabe - Sandmair - Marx

(56) Entgegenhaltungen:
- WO-A-2006/060181
- US-A1- 2003 023 190

## Beschreibung

Die Erfindung betrifft einen Katheter zur Verabreichung einer Substanz in ein Körpergewebe, insbesondere in Gehirnstrukturen. Solche Katheter werden beispielsweise in neurochirurgischen Verfahren, beispielsweise CED-Verfahren (convection enhanced delivery) durch die Schädeldecke in das Gehirngewebe eingebracht, um direkt im Gehirngewebe eine Substanz freizugeben.

Nachteilig bei bisherigen Kathetern ist, dass die einzubringende flüssige Substanz nach Verlassen des distalen Endes des Katheters aufgrund des Flüssigkeitsdrucks im Verabreichungsgebiet zwischen äußerer Katheterwand und dem daran angrenzenden Gewebe entlang des Katheters zurückfließt. Dieses Zurückfließen in den "Katheterschacht" wird auch Backflow genannt. Bei einer rechnergestützten Simulation der Flüssigkeitsverteilung im Gehirngewebe kann daher das distale Ende des Katheters nicht mehr als punktförmige Quelle idealisiert werden, da sich die Subtanz in unbestimmbarer Weise entlang des Katheters verteilt. Weiterhin besteht bei einem nahe der äußeren Gehirnhaut gesetzten Katheterende die Gefahr, dass die Substanz bei fehlender Abdichtung aus dem Gewebe durch den Katheterschacht wieder austritt. Dies gilt es unbedingt zu verhindern. Eine fest auf der äußeren Katheterwand angebrachte Abdichtung ist beim Einbringen des Katheters allerdings hinderlich, wenn nicht sogar schädlich, da hierdurch Gehirngewebe durch das Einführen einer rigiden Abdichtung verletzt werden könnte. Auch können durch hierdurch verursachte Schadstellen im Gehirngewebe Kanäle entstehen, durch welche die zu verabreichende Substanz aus der Verabreichungsgegend wieder entweichen kann. Daraus folgt ebenfalls eine unerwünschte Abweichung der realen Flüssigkeitsverteilung im Vergleich zur simulierten Flüssigkeitsverteilung, die eine punktförmige Quelle der Verabreichungssubstanz annimmt. Zu einer genauen Simulation ist das Begrenzen des Backflows auf eine minimale Weglänge notwendig.

Aus der US 2006/0116636 A1 ist ein Katheter bekannt, dessen distales Ende eine Ummantelung aufweist, welche ihr Volumen aufgrund des Vorhandenseins von Flüssigkeit vergrößert. Durch dieses "Aufquellen" soll eine Abdichtung zwischen Katheteraußenwand und dem daran angrenzenden Gehirngewebe erreicht werden. Im aufgequollenen Zustand weist der betroffene Bereich dieses Katheters jedoch wiederum eine glatte Außenwandung auf. So ist nicht sicherzustellen, dass die zu verabreichende Flüssigkeit nicht doch wieder zwischen dem aufgequollenen Bereich und dem in der Struktur unregelmäßigen Gehirngewebe durch feine Kanäle am Dichtbereich vorbei in Richtung des proximalen Endes des Katheters vordringt. Eine sichere Abdichtung ist daher nicht gegeben.

Ein derartiger Katheter, wie er im Oberbegriff nach Anspruch 1 beschrieben ist, auch aus dem Dokument US 2003/0023190 A1 bekannt.

Es ist Aufgabe der vorliegenden Erfindung, einen Katheter bereitzustellen, der eine optimierte Abdichtung zwischen Katheter und Gehirngewebe sicherstellt.

Diese Aufgabe wird durch einen Katheter gemäß dem Anspruch 1 gelöst. Die Unteransprüche definieren bevorzugte Ausführungsformen der Erfindung.

Ein erfindungsgemäßer Katheter zur Verabreichung einer Substanz in ein Körpergewebe, insbesondere in Gehirnstrukturen, hat einen länglichen Katheterkörper, der ein oder mehrere Lumen umgibt, wobei der Katheterkörper über einen oder mehrere Teilabschnitte seiner Länge ein Material aufweist, dessen Volumen sich aufgrund geänderter Umgebungsbedingungen in der Verabreichungsumgebung ändert, wobei durch die Volumenänderung eine profilierte Katheraußenfläche gebildet wird.

Mit anderen Worten bildet die Katheteraußenwand in definierten Bereichen beziehungsweise Teilabschnitten nach dem Einbringen des Katheters in Körpergewebe durch Volumenänderung eine Stuktur aus, was durch chemische oder physikalische Änderungen in unmittelbarer Umgebung des Katheterendes im Körpergewebe hervorgerufen wird. Durch einen derartig profilierten Katheter wird ein Zurückfließen der zu verabreichenden Substanz bzw. Flüssigkeit zwischen Katheteraußenwand und dem daran angrenzenden Körpergewebe wirkungsvoll verhindert.

So ist es bei einem solchen Katheter letztendlich möglich, bei guter Abdichtung des im Gehirngewebe gesetzten Katheters eine möglichst reibungsfreie und verletzungsfreie Einbringung des Katheters durch einen konstanten äußeren Querschnitt des Katheters zu gewährleisten.

Dabei ist es denkbar, dass der Katheter in besagten Teilabschnitten nur teilweise das volumenveränderliche Material, wie etwa ein biokompatibles Hydrogel-Polymer aufweist oder dort zur Gänze daraus besteht.

In einer bevorzugten Ausführungsform weist dieses Profil, beispielsweise ein Treppenstufenprofil, mehrere Erhebungen entlang der Katheterachse auf. Dadurch werden gleichzeitig mehrere Dichtabschnitte gebildet, wobei jeder Dichtabschnitt einzeln zwischen Katheteraußenwand und Gehirnstruktur abdichtet. Dies hat den Vorteil, dass bei einer eventuellen Fehlstelle und einem dadurch hervorgerufenen Ausfall eines Dichtabschnittes die dahinter in Richtung des proximalen Endes des Katheters liegenden Dichtabschnitte die zu verabreichende Substanz zurückhalten. Durch eine solche Ausgestaltung ergibt sich eine gewisse Redundanz bei der Abdichtung, welche deren Sicherheit erhöht.

Dabei ist es möglich, dass zwischen den Erhebungen, beispielsweise radial umlaufende Aussparungen oder Ausnehmungen bzw. vertiefte Abschnitte ausgebildet sind. Diese können "Sammelstellen" für die Substanz bilden und auch dadurch einen weiteren Rückfluss verhindern.

Weiterhin ist es möglich, dass das Profil innerhalb eines oder mehrerer Teilabschnitte ausgebildet wird oder in der Gesamtheit betrachtet ein Profil gebildet wird, welches sich über mehrere Teilabschnitte hinweg erstreckt. Dadurch sind vielfältige Ausgestaltungsmöglichkeiten möglich, die je nach Einsatzzweck und -ziel, beispielsweise in Gewebestrukturen verschiedener Materialeigenschaften eingesetzt werden können.

Auch ist es möglich, dass zwischen mehreren dieser Teilabschnitte wiederum Teilabschnitte ausgebildet sind, welche aus dem Material des eigentlichen Katheters oder zumindest aus einem anderen als dem volumenveränderlichen Material sind, welches in den Teilabschnitten vorliegt, aus dem die späteren Dichtabschnitte gebildet werden. Insbesondere das äußerste distale Ende des Katheters kann aus einem anderen Material als diese Teilabschnitte bestehen. Je kleiner dabei die Erstreckung des äußersten volumenveränderlichen distalen Endes des Katheters ist, desto näher kann das Katheterende in Bereiche unter der Gehirnhaut gesetzt werden, da der Backflow durch die unmittelbare Abdichtung noch im Gehirngewebe auf eine äußerst kurze Wegstrecke begrenzt ist. So kann ein Herausfließen der Substanz aus der Gehirnstruktur verhindert werden.

Um je nach Einsatzzweck und -ziel des Katheters verschiedene Profile zu ermöglichen, können die Teilabschnitte und/oder die Bereiche zwischen den Teilabschnitten unterschiedliche Abmessungen entlang der Katheterlängsachse aufweisen. So ist insbesondere auch ein Katheter denkbar, welcher einen Abschnitt aufweist, der sich über die gesamte Länge des Katheters erstreckt.

Ferner ist es auch denkbar, dass einer oder mehrere der Teilabschnitte in sich selbst unterschiedliche Volumenänderungen erfährt bzw. erfahren. Ebenso können auch verschiedene Teilabschnitte verschieden große Volumenänderungen erfahren. Dies kann beispielsweise durch unterschiedlich große Anhäufungen von dem volumenveränderlichen Material in den Teilabschnitten bewirkt werden oder durch Verwendung unterschiedlicher Materialien oder Materialzusammensetzungen, welche eine unterschiedliche Volumenzunahme aufweisen.

In einer weiteren Ausführungsform kann auf der Katheteraußenwand ein Profil gebildet werden, welches nicht spiegelsymmetrisch zu einer Ebene durch die Katheterlängsachse ist. Das kann beispielsweise ein schraubenähnliches Profil sein. Dadurch lässt sich trotz einer guten Abdichtung der Katheter nach Gebrauch aus dem Gehirngewebe "ausschrauben", ohne dass dabei anliegendes Gehirngewebe geschädigt wird, wie das beim Herausziehen symmetrischer Profile der Fall sein könnte.

Die Volumenänderung des Materials kann dadurch bewirkt werden, dass physikalische oder chemische Umgebungsbedingungen bzw. Einflussfaktoren in der Umgebung des Materials sich ändern oder geändert werden und dieses Material dadurch eine Volumenänderung erfährt. Dies können insbesondere elektrische Spannungs- und/oder Stromänderungen, pH-Wert-Änderungen, Änderungen in der Konzentration von Ionen, Änderungen einer Lichtstärke und/oder Lichtwellenlänge, Änderungen im Vorhandensein von Wasser, Änderungen im elektrischen Feld, Änderungen in der Temperatur oder Änderungen in der Konzentration von chemischen Elementen und/oder Verbindungen oder eine Kombination davon sein. Bevorzugterweise können es Änderungen sein, die aufgrund des Vohandenseins körpereigener Umgebungsbedingungen in der Verabreichungsumgebung hervorgerufen werden. Auch können es Änderungen sein, die durch das Vorhandensein der zu verabreichenden Substanz hervorgerufen werden, beispielsweise beim Einbringen der Substanz durch den Katheter.

Die Erfindung wird im Weiteren anhand der beiliegenden Zeichnungen näher erläutert. Sie kann alle hierin beschriebenen Merkmale einzeln sowie in jedweder sinnvollen Kombination umfassen. In den Zeichnungen zeigen:
- Figur 1: verschiedene Ausführungformen eines Katheters, sowohl in der Ausgangsform vor der Volumenänderung als auch in der Endform nach der Volumenänderung;
- Figur 2: ein distales Ende eines Katheters sowohl in der Ausgangsform vor der Volumenänderung als auch in der Endform nach der Volumenänderung.

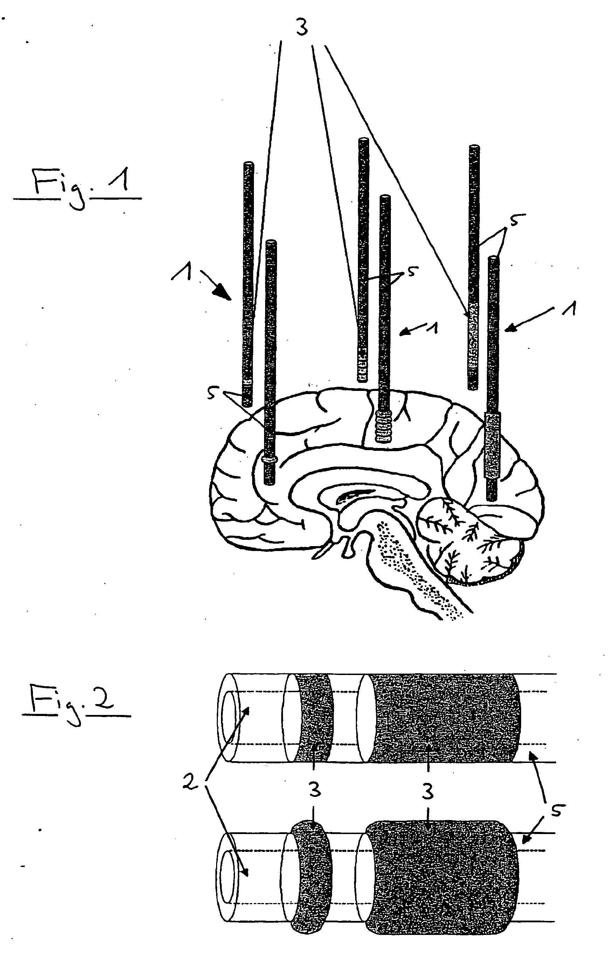

Die in Figur 1 gezeigten Katheter 1 weisen einen Katheterkörper 5 mit einem proximalen Ende und einem distalen Ende auf. Für Verabreichung einer flüssigen Substanz in ein Körpergewebe, wie hier beispielsweise ein Gehirngewebe, wird das distale Ende des Katheterkörpers 5 durch die Schädeldecke in die Gehirnstruktur zur gewünschten Verabreichungsstelle eingebracht. Danach wird die zu verabreichende Substanz vom proximalen Ende durch den Katheterkörper 5 zu dessen distalen Ende durch beispielsweise Überdruck am proximalen Ende des Katheters 1 in die Gehirnstruktur eingebracht.

Durch gerade diesen Überdruck, welcher den Transport der zu verabreichenden Substanz ermöglicht, ist die zu verabreichende Substanz bestrebt, vom Verabreichungsort aus über den entstandenen Katheterschacht im Gehimgewebe zurückzufließen und ggfs. die Gehirnstruktur wieder zu verlassen. Dabei ist selbst ein Zurückfließen über eine relativ kurze Wegstrecke entlang des Katheterkörpers möglichst zu vermeiden, da bei rechnergestützten Simulationen der Flüssigkeitsverteilung im Gehimgewebe ein definierter Einbringungsort der zu verabreichenden Substanz nötig ist. Bei einer undefinierten Verteilung der Substanz im Katheterschacht wäre dies nicht möglich.

So wird beim erfindungsgemäßen Katheter dieses Zurückfließen bzw. der Backflow dadurch verhindert, dass am Katheterkörper 5 Dichtelemente vorgesehen werden, wie in den Figuren 1 und 2 aufgezeigt wird.

Um trotzdem den Katheter in die Gehirnstruktur einbringen zu können, ohne durch vom Querschnitt des Katheterkörpers 5 abstehende Dichtelemente Verletzungen am Gehirngewebe oder potentielle Rücklaufkanäle hervorzurufen, sind am Katheterkörper 5 ein oder mehrere Teilabschnitte 3 angeordnet. Diese Teilabschnitte 3 weisen dabei ein Material auf, welches gegenüber geänderten Umgebungsbedingungen sensibel ist. Diese Sensibilität ruft eine Volumenzunahme der besagten Teilabschnitte 3 hervor, wodurch nach Einbringen des Katheters 1 in die Gehirnstruktur Dichtelemente gebildet werden. Dabei sollten diese Teilabschnitte 3 nach Möglichkeit nahe dem distalen Ende des Katheterkörpers 5 ausgebildet sein, um den Backflow der zu verabreichenden Substanz auf eine möglichst kurze Wegstrecke zu begrenzen. Dabei können diese Teilabschnitte 3 in sich oder global betrachtet untereinander eine definierte Struktur bzw. ein Profil ausbilden, was die Abdichtung zwischen Katheterkörper 5 und Gehirnstruktur weiter verbessert. Drei Ausgestaltungsmöglichkeiten sind dabei in Figur 1 ersichtlich. Weiterhin ist gezeigt, wie sich die Teilabschnitte, ausgehend von einem konstanten Querschnitt des Katheterkörpers 5, nach dem Einbringen in die Gehirnstruktur an Volumen zunehmen und dadurch Dichtelemente entstehen ohne vorher beim Einbringen des Katheterkörpers 5 hinderlich zu sein.

Dabei kann sich ein Teilabschnitt 3 auch über die Gehirnstruktur hinaus erstrecken. Dies hat insbesondere den Vorteil, dass auch bei einem Verabreichungsort nahe der äußeren Gehirnhaut der Katheterschacht zuverlässig abgedichtet werden kann.

Das in Figur 2 gezeigte distale Katheterende umfasst zwei Teilabschnitte 3 mit volumenveränderlichem Material. Zwischen diesen Teilabschnitten 3 erstrecken sich wiederum Teilabschnitte aus einem anderen Material, nämlich dem Material des eigentlichen Katheters, ebenso wie das äußerste distale Ende des Katheters, an dem letztendlich die zu verabreichende Substanz austritt.

Der Katheterkörper 5 umschließt in der hier gezeigten Ausführungsform ein Lumen 2, durch welches die zu verabreichende Substanz in das Gehimgewebe eingebracht wird. Weiterhin ist in der unteren Darstellung der Figur 2 zu sehen, wie sich das Volumen der Teilabschnitte 3 im Endzustand, nämlich nach dem Setzen des Katheters in die Gehirnstruktur vergrößert und dadurch den Raum zwischen Katheteraußenwand und Gehirnstruktur abdichtet. Eine solche Ausgestaltungsform hat weiterhin den Vorteil, dass bei der Tendenz des Zurückfließens der Substanz diese durch mehrere an den Teilabschnitten 3 gebildete redundante Dichtungen daran gehindert wird. Selbst wenn die Substanz den vom distalen Ende aus gesehen ersten Teilabschnitt überwindet und dabei Energie dissipiert, tritt sie daraufhin in einen zwischen den zwei Teilabschnitten 3 gebildeten "Sammelraum". Diese Aneinanderreihung von Strömungshindernissen ist dabei beliebig oft wiederholbar, so dass letztendlich der Rückfluss der zu verabreichenden Substanz auf ein Minimum reduziert wird. Verstärkt wird dieser Effekt noch dadurch, dass die Teilabschnitte 3 je nach Anforderung verschiedene Längen aufweisen können, um so verschieden lange Dichtungen zu bilden.

## Patentansprüche

1. Katheter (1) zur Verabreichung einer Substanz in ein Körpergewebe, insbesondere in Gehimstrukturen, mit einem länglichen Katheterkörper (5), der ein oder mehrere Lumen (2) umgibt, wobei der Katheterkörper (5) über einen oder mehrere Teilabschnitte (3) seiner Länge ein Material aufweist, dessen Volumen sich aufgrund geänderter Umgebungsbedingungen in der Verabreichungsumgebung ändert, **dadurch gekennzeichnet, dass** durch die Volumenänderung eine profilierte Katheteraußenfläche mit mehreren Dichtabschnitten gebildet wird.

2. Katheter nach Anspruch 1, **dadurch gekennzeichnet, dass** das Profil mehrere Erhebungen entlang der Katheterlänge umfasst.

3. Katheter nach Anspruch 2, **dadurch gekennzeichnet, dass** zwischen den Erhebungen nicht erhobene Abschnitte oder vertiefte Abschnitte ausgebildet sind.

4. Katheter nach einem der Ansprüche 1 bis 3, **dadurch gekennzeichnet, dass** das Profil auf einem Teilabschnitt selbst und/oder über mehrerer Teilabschnitte hinweg gebildet wird.

5. Katheter nach einem der Ansprüche 1 bis 4, **dadurch gekennzeichnet, dass** sich zwischen mehreren beabstandeten Teilabschnitten (3) Teilabschnitte aus dem Kathetermaterial oder einem anderen Material als dem der Teilabschnitte (3) erstrecken, insbesondere auch am distalen Ende des Katheterkörpers (5).

6. Katheter nach Anspruch 1 bis 5, **dadurch gekennzeichnet, dass** die Teilabschnitte (3) und/oder die Teilabschnitte zwischen den Teilabschnitten (3) untereinander gleiche oder unterschiedliche Abmessungen aufweisen.

7. Katheter nach einem der Ansprüche 1 bis 4, **dadurch gekennzeichnet, dass** sich ein Teilabschnitt (3) über die gesamte Länge des Katheters (1) erstreckt.

8. Katheter nach einem der Ansprüche 1 bis 7, **dadurch gekennzeichnet, dass** der oder die Teilabschnitte (3) selbst oder untereinander unterschiedliche Volumenänderungen erfahren, insbesondere durch unterschiedliche Ausgangsvolumina des Materials und/oder unterschiedliche Materialien/Materialzusammensetzungen in dem oder den Teilabschnitten (3).

9. Katheter nach einem der Ansprüche 1 bis 8, **dadurch gekennzeichnet, dass** der oder die Teilabschnitte (3) eine zu einer Ebene durch die Katheterlängsachse nicht spiegelsymmetrische Struktur am Katheterkörper (5) ausbilden, insbesondere eine schraubenähnliche Struktur.

10. Katheter nach einem der Ansprüche 1 bis 9, **dadurch gekennzeichnet, dass** das Material ein gegenüber physikalischen oder chemischen Einflussfaktoren volumensensibles Material ist.

11. Katheter nach einem der Ansprüche 1 bis 10, **dadurch gekennzeichnet, dass** das Material ein gegenüber einem oder mehrerer der folgenden Einflussfaktoren sensibles Material ist:
- elektrischen Spannungs- und/oder Stromänderungen
- pH-Wert-Änderungen
- Änderungen in der Konzentration von Ionen
- Änderungen im Vorhandensein von Wasser
- Änderungen im elektromagnetischen Feld
- Temperaturänderungen
- Änderungen in der Konzentration von chemischen Elementen oder Verbindungen
- Änderungen einer Lichtstärke und/oder Lichtwellenlänge.

12. Katheter nach einem der Ansprüche 1 bis 11, **dadurch gekennzeichnet, dass** das Material ein gegenüber körpereigenen Umgebungsbedingungen in der Verabreichungsumgebung sensibles Material ist.

13. Katheter nach einem der Ansprüche 1 bis 12, **dadurch gekennzeichnet, dass** das Material ein gegenüber der zu verabreichenden Substanz sensibles Material ist.

## Claims

1. A catheter (1) for administering a substance into a body tissue, in particular into brain structures, comprising an elongated catheter body (5) which surrounds one or more lumens (2), wherein one or more partial portions (3) of the length of the catheter body (5) comprise a material which changes its volume due to changes in the ambient conditions in the administering environment, **characterised in that** a profiled outer surface of the catheter comprising a plurality of sealing portions is formed by the change in volume.

2. The catheter according to claim 1, **characterised in that** the profile includes a plurality of bumps along the length of the catheter.

3. The catheter according to claim 2, **characterised in that** non-raised or recessed portions are formed between the bumps.

4. The catheter according to any one of claims 1 to 3, **characterised in that** the profile is formed on a partial portion itself and/or over and beyond a plurality of partial portions.

5. The catheter according to any one of claims 1 to 4, **characterised in that** extending between a plurality of spaced partial portions (3) are partial portions which are made of the material of the catheter or a material other than that of the partial portions (3), in particular also at the distal end of the catheter body (5).

6. The catheter according to any one of claims 1 to 5, **characterised in that** the partial portions (3) and/or the partial portions between the partial portions (3) exhibit identical or different dimensions to each other.

7. The catheter according to any one of claims 1 to 4, **characterised in that** a partial portion (3) extends over the entire length of the catheter (1).

8. The catheter according to any one of claims 1 to 7, **characterised in that** the partial portion or portions (3) experience different changes in volume within themselves or between each other, in particular due to different initial volumes of the material and/or different materials/material compositions in the partial portion or portions (3).

9. The catheter according to any one of claims 1 to 8, **characterised in that** the partial portion or portions (3) form a structure on the catheter body (5) which does not have mirror symmetry with a plane through the longitudinal axis of the catheter, in particular a screw-like structure.

10. The catheter according to any one of claims 1 to 9, **characterised in that** the material is a material which is sensitive in its volume to physical or chemical influencing factors.

11. The catheter according to any one of claims 1 to 10, **characterised in that** the material is a material which is sensitive to one or more of the following influencing factors:
- changes in voltage and/or current;
- changes in pH value;
- changes in the concentration of ions;
- changes in the presence of water;
- changes in the electromagnetic field;
- changes in temperature;
- changes in the concentration of chemical elements or compounds;
- changes in light intensity and/or light wavelength.

12. The catheter according to any one of claims 1 to 11, **characterised in that** the material is a material which is sensitive to bodily ambient conditions in the administering environment.

13. The catheter according to any one of claims 1 to 12, **characterised in that** the material is a material which is sensitive to the substance to be administered.

## Revendications

1. Cathéter (1) pour administrer une substance dans un tissu corporel, en particulier dans des structures du cerveau, avec un corps de cathéter (5) oblong qui entoure un ou plusieurs conduits (2), le corps de cathéter (5) présentant, sur un ou plusieurs segments partiels (3) de sa longueur, un matériau dont le volume varie en raison de conditions ambiantes modifiées dans le milieu d'administration, **caractérisé en ce que** du fait de la variation de volume il est formé une surface extérieure de cathéter profilée avec plusieurs segments d'étanchéité.

2. Cathéter selon la revendication 1, **caractérisé en ce que** le profil comprend plusieurs reliefs le long de la longueur du cathéter.

3. Cathéter selon la revendication 2, **caractérisé en ce que** des segments non en relief ou des segments en creux sont réalisés entre les reliefs.

4. Cathéter selon l'une quelconque des revendications 1 à 3, **caractérisé en ce que** le profil est formé sur un segment partiel lui-même et/ou sur plusieurs segments partiels.

5. Cathéter selon l'une quelconque des revendications 1 à 4, **caractérisé en ce que** des segments partiels dans le matériau du cathéter ou dans un autre matériau que celui des segments partiels (3) s'étendent entre plusieurs segments partiels (3) espacés, en particulier aussi à l'extrémité distale du corps (5) du cathéter.

6. Cathéter selon l'une quelconque des revendications 1 à 5, **caractérisé en ce que** les segments partiels (3) et/ou les segments partiels entre les segments partiels (3) présentent des dimensions identiques ou différentes.

7. Cathéter selon l'une quelconque des revendications 1 à 4, **caractérisé en ce qu'**un segment partiel (3) s'étend sur toute la longueur du cathéter (1).

8. Cathéter selon l'une quelconque des revendications 1 à 7, **caractérisé en ce que** le ou les segments partiels (3) subissent des variations de volume identiques ou différentes les unes des autres, en particulier du fait de volumes initiaux différents du matériau et/ou de matériaux/compositions de matériau différents dans le ou les segments partiels (3).

9. Cathéter selon l'une quelconque des revendications 1 à 8, **caractérisé en ce que** le ou les segments partiels (3) réalisent, sur le corps (5) du cathéter, une structure qui ne présente pas une symétrie en miroir par rapport à un plan passant par l'axe longitudinal du cathéter, en particulier une structure en hélice.

10. Cathéter selon l'une quelconque des revendications 1 à 9, **caractérisé en ce que** le matériau est un matériau dont le volume est sensible aux facteurs d'influence physiques ou chimiques.

11. Cathéter selon l'une quelconque des revendications 1 à 10, **caractérisé en ce que** le matériau est un matériau sensible à l'égard d'un ou de plusieurs des facteurs d'influence suivantes :
- variations de tension et/ou d'intensité électrique
- variations du pH
- variations de la concentration en ions
- variations de la teneur en eau
- variations du champ électromagnétique
- variations de la température
- variations de la concentration d'éléments ou de composés chimiques
- variations d'intensité lumineuse et/ou de longueur d'onde lumineuse.

12. Cathéter selon l'une quelconque des revendications 1 à 11, **caractérisé en ce que** le matériau est un matériau sensible à l'égard de conditions ambiantes corporelles dans le milieu d'administration.

13. Cathéter selon l'une quelconque des revendications 1 à 12, **caractérisé en ce que** le matériau est un matériau sensible à l'égard de la substance à administrer.
